# EUROPEAN PATENT SPECIFICATION

(11) **EP 0 573 329 B1**
(45) Date of publication and mention of the grant of the patent: **02.07.1997**
(21) Application number: 93401341.8
(22) Date of filing: 25.05.1993
(51) Int. Cl.: C11D 1/94, C11D 17/00, A61K 7/08, A61K 7/50

(54) **High foaming nonionic surfactant based liquid detergent**
Flüssiges Reinigungsmittel auf der Basis von starkschäumenden, nichtionischen, oberflächenaktiven Mitteln
Composition détergente liquide à base de surfactant non ionique très moussant

(30) Priority: 03.06.1992 US 893132
(43) Date of publication of application: 08.12.1993
(73) Proprietor: Colgate-Palmolive Company, New York, N.Y. 10022-7499 (US)
(72) Inventor: Repinec, Stephen T., New Jersey 08822 (US); Gomes, Gilbert S., Somerset, New Jersey (US); Erilli, Rita, B-4000 Rocourt (BE)
(74) Representative: Le Guen, Gérard

(56) References cited:
- EP-A- 0 155 737
- EP-A- 0 208 440
- WO-A-91/00138
- US-A- 3 950 417
- US-A- 4 595 526
- US-A- 4 992 107

## Description

### BACKGROUND OF THE INVENTION

The present invention relates to novel light duty liquid detergent compositions with high foaming properties, containing a nonionic surfactant as the major active ingredient supplemented with lesser amounts of a specific group of anionic surfactants and even smaller amounts of a Zwitterionic betaine surfactant in an aqueous medium.

Nonionic surfactants are in general chemically inert and stable toward pH change and are therefore well suited for mixing and formulation with other materials. The superior performance of nonionic surfactants on the removal of oily soil is well recognized. Nonionic surfactants are also known to be mild to human skin. However, as a class, nonionic surfactants are known to be low or moderate foamers. Consequently, for detergents which require copious and stable foam, the application of nonionic surfactants is limited. There have been substantial interest and efforts to develop a high foaming detergent with nonionic surfactants as the major ingredient. Yet, little has been achieved.

The prior art is replete with light duty liquid detergent compositions containing nonionic surfactants in combination with anionic and/or betaine surfactants wherein the nonionic detergent is not the major active surfactant, as shown in U.S. Patent No. 3,658,985 wherein an anionic based shampoo contains a minor amount of a fatty acid alkanolamide. U.S. Patent No. 3,769,398 discloses a betaine-based shampoo containing minor amounts of nonionic surfactants. This patent states that the low foaming properties of nonionic detergents renders its use in shampoo compositions non-preferred. U.S. Patent No. 4,329,335 also discloses a shampoo containing a betaine surfactant as the major ingredient and minor amounts of a nonionic surfactant and of a fatty acid mono- or di-ethanolamide. U.S. Patent No. 4,259,204 discloses a shampoo comprising 0.8-20% by weight of an anionic phosphoric acid ester and one additional surfactant which may be either anionic, amphoteric, or nonionic. U.S. Patent No. 4,329,334 discloses an anionic-amphoteric based shampoo containing a major amount of anionic surfactant and lesser amounts of a betaine and nonionic surfactants.

U.S. Patent No. 3,935,129 discloses a liquid cleaning composition based on the alkali metal silicate content and containing five basic ingredients, namely, urea, glycerin, triethanolamine, an anionic detergent and a nonionic detergent. The silicate content determines the amount of anionic and/or nonionic detergent in the liquid cleaning composition. However, the foaming property of these detergent compositions is not discussed therein.

U.S. Patent No. 4,129,515 discloses a heavy duty liquid detergent for laundering fabrics comprising a mixture of substantially equal amounts of anionic and nonionic surfactants alkanolamines and magnesium salts, and, optionally, zwitterionic surfactants as suds modifiers.

U.S. Patent No. 4,224,195 discloses an aqueous detergent composition for laundering socks or stockings comprising a specific group of nonionic detergents, namely, an ethylene oxide of a secondary alcohol, a specific group of anionic detergents, namely, a sulfuric ester salt of an ethylene oxide adduct of a secondary alcohol, and an amphoteric surfactant which may be a betaine, wherein either the anionic or nonionic surfactant may be the major ingredient. The specific class of anionics utilized in this patent is the very same group of anionic detergents expressly excluded in present invention in order to eliminate the alkanol ethoxylate sulfation process and the potential dioxane toxicity problem. Furthermore, this patent finds heavily foaming detergents undesirable for the purpose of washing socks.

The prior art also discloses detergent compositions containing all nonionic surfactants as shown in U.S. Patent Nos. 4,154,706 and 4,329,336 wherein the shampoo compositions contain a plurality of particular nonionic surfactants in order to effect desirable foaming and detersive properties despite the fact that nonionic surfactants are usually deficient in such properties.

U.S. Patent No. 4,013,787 discloses a piperazine based polymer in conditioning and shampoo compositions which may contain all nonionic surfactant or all anionic surfactant.

U.S. Patent No. 4,450,091 discloses high viscosity shampoo compositions containing a blend of an amphoteric betaine surfactant, a polyoxybutylenepolyoxyethylene nonionic detergent, an anionic surfactant, a fatty acid alkanolamide and a polyoxyalkylene glycol fatty ester. But, none of the exemplified compositions contains an active ingredient mixture wherein the nonionic detergent is present in major proportion, probably due to the low foaming properties of the polyoxybutylene polyoxyethylene nonionic detergent.

U.S. Patent No. 4,595,526 describes a composition comprising a nonionic surfactant, a betaine surfactant, an anionic surfacant and a C₁₂-C₁₄ fatty acid monethanolamide foam stabilizer.

However, none of the above-cited patents discloses a high foaming, nonionic based, liquid detergent composition containing a nonionic surfactant as a major active ingredient and minor amounts of a supplementary high foaming anionic sulfate or sulfonate surfactant excluding ethoxylated alcohol ether sulfates, a supplementary foaming zwitterionic surfactant selected from betaine type surfactants, an effective amount of an alkyl monoethanol amide and an effective amount of an alkyl diethanol amide as the essential ingredients, wherein the nonionic ingredient constitutes more than 50% of the total surfactant content and the composition does not contain any amine oxide, formate on HETDA type compounds and the concentration of the zwitterionic is less than or equal to 2.5 wt.%.

### SUMMARY OF THE INVENTION

It has now been found that a high foaming liquid detergent can be formulated with a nonionic surfactant as the major active ingredient which has desirable cleaning properties, mildness to the human skin and avoids the dioxane toxicity problem associated with the sulfation process of manufacturing anionic ethoxylated alcohol ether sulfates.

Accordingly, one object of the invention is to provide novel, high foaming, nonionic based, light duty liquid detergent compositions containing a nonionic ionic surfactant at a concentration of at least 50% of the total surfactant content.

Another object of this invention is to provide novel, nonionic based, liquid detergent compositions containing a major amount of nonionic surfactant supplemented with lesser amounts of an anionic surfactant and 10 wt. percent or less of a zwitterionic betaine surfactant wherein the composition does not contain a formate, amine oxide or HETDA.

Still another object of this invention is to provide a novel, nonionic based, liquid detergent with desirable high foaming and cleaning properties which is mild to the human skin.

A further object of this invention is to provide a novel, nonionic based liquid detergent containing a supplemental anionic surfactant excluding the ethoxylated alkyl ether sulfates which eliminates the alkanol ethoxylate sulfation process and the potential dioxane toxicity problem.

Additional objects, advantages and novel features of the invention will be set forth in part in the description which follows, and in part will become apparent to those skilled in the art upon examination of the following or may be learned by practice of the invention. The objects and advantages of the invention may be realized and attained by means of the instrumentalities and combinations particularly pointed out in the appended claims.

To achieve the foregoing and other objects and in accordance with the purpose of the present invention, as embodied and broadly described herein the novel, high foaming, nonionic based, light duty liquid detergent of this invention comprises three essential surfactants a water soluble, ethoxylated, nonionic surfactant as the major active ingredient in an amount exceeding 50% by weight of the total surfactant content; a supplemental amount of a foaming anionic surfactant selected from the group consisting of water soluble organic sulfates and organic sulfonates, excluding the ethoxylated alkyl ether sulfates; and 10 wt.% or less of a zwitterionic surfactant selected from the class of betaines dissolved in an aqueous vehicle, wherein the composition does not contain any amine oxide, formate or HETDA ingredients.

More specifically, the present invention relates to a high foaming, nonionic based, liquid detergent containing more than 50% by weight of the total surfactant content of a nonionic surfactant selected from the group consisting of water soluble primary aliphatic alcohol ethoxylates secondary aliphatic alcohol ethoxylates, alkyl phenol ethoxylates and alcohol ethylene oxide propylene oxide condensates; and supplementary amounts of an anionic surfactant selected from the group consisting of water soluble salts of C₈-C₁₈, alkyl sulfates, C₈-C₁₆ benzene sulfonates, C₁₀-C₂₀ paraffin sulfonates, alpha C₁₀-C₂₄ olefin sulfonates, C₈-C₁₈ alkyl suloacetates, C₈-C₁₈ alkyl sulfosuccinate esters, C₈-C₁₈ acyl isethionates and C₈-C₁₈ acyl taurates; and 2.5 wt.% or less of a water soluble zwitterionic betaine surfactant; the total content of said supplementary surfactants, constituting less that 50% by weight of the total surfactant content, dissolved in an aqueous vehicle.

This particular combination of three ingredients in the proportions, by weight, of more than 50% of the nonionic surfactant of the total surfactant content and 15 to 48% of the sum of anionic surfactant and betaine surfactant wherein the concentration of the betaine surfacant is 10 wt. percent or less, is critical to the high foaming and desirable cleansing properties of present liquid detergent and the retention of the mildness to the skin property. The total amount of surfactants may constitute 10%-55%, preferably 20%-40%, most preferably 25%-35%, by weight of the liquid composition.

### DETAILED DESCRIPTION OF THE INVENTION

The nonionic surfactant which constitutes the major ingredient in present liquid detergent is present in amounts of 10%-30%, preferably 13%-25%, most preferably 16%-22%, by weight of the composition and provides superior performance in the removal of oily soil and mildness to human skin.

The water soluble nonionic surfactants utilized in this invention are commercially well known and include the primary aliphatic alcohol ethoxylates, secondary aliphatic alcohol ethoxylates, alkylphenol ethoxylates and ethylene-oxide-propylene oxide condensates on primary alkanols, such a Plurafacs® (BASF) and condensates of ethylene oxide with sorbitan fatty acid esters such as the Tweens® (ICI). The nonionic synthetic organic detergents generally are the condensation products of an organic aliphatic or alkyl aromatic hydrophobic compound and hydrophilic ethylene oxide groups. Practically any hydrophobic compound having a carboxy, hydroxy, amido, or amino group with a free hydrogen attached to the nitrogen can be condensed with ethylene oxide or with the polyhydration product thereof, polyethylene glycol, to form a water-soluble nonionic detergent. Further, the length of the polyethenoxy chain can be adjusted to achieve the desired balance between the hydrophobic and hydrophilic elements.

The nonionic detergent class includes the condensation products of a higher alcohol (e.g., an alkanol containing 8 to 18 carbon atoms in a straight or branched chain configuration) condensed with 5 to 30 moles of ethylene oxide, for example, laurylmyristyl alcohol condensed with 16 moles of ethylene oxide (EO), tridecanol condensed with 6 moles of EO, myristyl alcohol condensed with 10 moles of EO per mole of myristyl alcohol, the condensation product of EO with a hear-cut of coconut fatty alcohol containing a mixture of fatty alcohols with alkyl chains varying from 10 to 14 carbon atoms in length and wherein the condensate contains either about 6 moles of EO per mole of total alcohol or about 9 moles of EO per mole of alcohol and tallow alcohol ethoxylates containing 6 EO to 11 EO per mole of alcohol.

A preferred group of the foregoing nonionic surfactants are the Neodol® ethoxylates (Shell Co.), which are higher aliphatic, primary alcohol containing 9-15 carbon atoms, such as C₉-C₁₁ alkanol condensed with 8 moles of ethylene oxide (Neodol® 91-8), C₁₂₋₁₃ alkanol condensed with 6.5 moles ethylene oxide (Neoldol® 23-6.5), C₁₂₋₁₅ alkanol condensed with 12 moles ethylene oxide (Neodol® 25-12), C₁₄₋₁₅ alkanol condensed with 13 moles ethylene oxide (Neodol® 45-13), and the like. Such ethoxamers have an HLB (hydrophobic lipophilic balance) value of 8-15 and give good/W emulsification, whereas ethoxamers with HLB values below 8 contain less than 5 ethyleneoxy groups and tend to be poor emulsifiers and poor detergents.

Additional satisfactory water soluble alcohol ethylene oxide condensates are the condensation products of a secondary aliphatic alcohol containing 8 to 18 carbon atoms in a straight or branched chain configuration condensed with 5 to 30 moles of ethylene oxide. Examples of commercially available nonionic detergents of the foregoing type are C₁₁-C₁₅ secondary alkanol condensed with either 9 EO (Tergitol® 15-S-9) or 12 EO (Tergitol® 15-S-12) marketed by Union Carbide.

Other suitable nonionic detergents include the polyethylene oxide condensates of one mole of alkyl phenol containing from 8 to 18 carbon atoms in a straight- or branched chain alkyl group with 5 to 30 moles of ethylene oxide. Specific examples of alkyl phenol ethoxylates include nonyl condensed with about 9.5 moles of EO per mole of nonyl phenol, dinonyl phenol condensed with about 12 moles of EO per mole of phenol, dinonyl phenol condensed with about 15 moles of EO per mole of phenol and di-isoctylphenol condensed with about 15 moles of EO per mole of phenol. Commercially available nonionic surfactants of this type include Igepal® CO-630 (nonyl phenol ethoxylate) marketed by GAF Corporation.

Also among the satisfactory nonionic detergents are the water-soluble condensation products of a C₈-C₂₀ alkanol with a heteric mixture of ethylene oxide and propylene oxide wherein the weight ratio of ethylene oxide to propylene oxide is from 2.5:1 to 4:1, preferably 2.8:1-3.3:1, with the total of the ethylene oxide and propylene oxide (including the terminal ethanol or propanol group) being from 60-85%, preferably 70-80%, by weight. Such detergents are commercially available from BASF-Wyandotte and a particularly preferred detergent is a C₁₀-C₁₆ alkanol condensate with ethylene oxide and propylene oxide, the weight ratio of ethylene oxide to propylene oxide being 3:1 and the total alkoxy content being about 75% by weight.

Condensates of 2 to 30 moles of ethylene oxide with sorbitan mono- and tri-C₁₀-C₂₀ alkanoic acid esters having a HLB of 8 to 15 also may be employed as the nonionic detergent ingredient in the described shampoo. These surfactants are well known and are available from Imperial Chemical Industries under the Tween trade name. Suitable surfactants include polyoxyethylene (4) sorbitan monolaurate, polyoxyethylene (4) sorbitan monostearate, polyoxyethylene (20) sorbitan trioleate and polyoxyethylene (20) sorbitan tristearate.

Other suitable water-soluble nonionic detergents which are less preferred are marketed under the trade name "Pluronics®." The compounds are formed by condensing ethylene oxide with a hydrophobic base formed by the condensation of propylene oxide with propylene glycol. The molecular weight of the hydrophobic portion of the molecule is of the order of 950 to 4000 and preferably 200 to 2,500. The addition of polyoxyethylene radicals to the hydrophobic portion tends to increase the solubility of the molecule as a whole so as to make the surfactant water-soluble. The molecular weight of the block polymers varies from 1,000 to 15,000 and the polyethylene oxide content may comprise 20% to 80% by weight. Preferably, these surfactants will be in liquid form and satisfactory surfactants are available as grades L 62 and L 64. Another suitable nonionic surfacants Lauropol 0207® sold by Witco Chemico, Synperionic® 9-11 sold by ICI Europe and Lialet 111® solb by Enichem.

The anionic surfactant, which is an essential ingredient of present liquid detergent composition, constitutes 1% to 10%, preferably 2%-9%, most preferably 3%-8%, by weight thereof and provides good foaming properties. However, preferably reduced amounts are utilized in order to enhance the mildness of the skin property desired in the inventive compositions, and thus, the weight ratio of nonionic detergent to anionic should exceed 3:1. In addition, the particular group of anionic surfactants utilized excludes the C₈-C₁₈ alkyl polyethenoxy ether sulfate surfactants in order to avoid the dioxane toxicity associated with the process of sulfation of ethoxylated alcohols. Thus, the ethoxylated alcohol ether sulfates are expressly excluded from the specific group of anionic surfactants utilized.

The anionic surfactants which may be used in the nonionic based liquid detergent of this invention are water soluble such as triethanolamine and include the sodium, potassium, ammonium and ethanolammonium salts of C₈-C₁₈ alkyl sulfates such as lauryl sulfate, myristyl sulfate and the like; linear C₈-C₁₆ alkyl benzene sulfonates; C₁₀-C₂₀ paraffin sulfonates; alpha olefin sulfonates containing 10-24 carbon atoms; C₈-C₁₈ alkyl sulfoacetates; C₈-C₁₈ alkyl sulfosuccinate esters; C₈-C₁₈ acyl isethionates; and C₈C-₁₈ acyl taurates. Preferred anionic surfactants are the water soluble C₁₂-C₁₆ alkyl sulfates, the C₁₀-C₁₅ alkylbenzene sulfonates, the C₁₃-C₁₇ paraffin sulfonates and the alpha C₁₂-C₁₈ olefin sulfonates.

The water-soluble zwitterionic surfactant, which is also an essential ingredient of present liquid detergent composition, constitutes 0.5-10%, preferably 2%-9%, most preferably 7%-8%, by weight and provides good foaming properties and mildness to the present nonionic based liquid detergent. The zwitterionic surfactant is a water soluble betaine having the general formula: 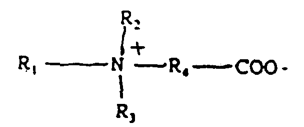 wherein R₁ is an alkyl group having 10 to 20 carbon atoms, preferably 12 to 16 carbon atoms, or the amido radical: 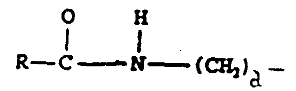 wherein R is an alkyl group having 9 to 19 carbon atoms and a is the integer 1 to 4; R₂ and R₃ are each alkyl groups having 1 to 3 carbons and preferably 1 carbon; R₄ is an alkylene or hydroxyalkylene group having from 1 to 4 carbon atoms and, optionally, one hydroxyl group. Typical alkyldimethyl betaines include decyl dimethyl betaine or 2-(N-decyl-N, N-dimethylammonia) acetate, coco dimethyl betaine or 2-(N-coco N, N-dimethylammonio) acetate, myristyl dimethyl betaine, palmityl dimethyl betaine, lauryl diemthyl betaine, cetyl dimethyl betaine, stearyl dimethyl betaine, etc. The amidobetaines similarly include cocoamidoethylbetaine, cocoamidopropyl betaine and the like. A preferred betaine is coco (C₈-C₁₈) amidopropyl dimethyl betaine, Two preferred betaine surfactants are Rewoteric® AMB 13 and Golmschmidt Betaine L7®.

All of the aforesaid three ingredients in this light duty liquid detergent are water soluble or water dispersible and remain so during storage.

This particular combination of anionic surfactant and betaine surfactant, provides a detergent system which coacts with the nonionic surfactant to product a liquid detergent composition with desirable foaming, foam stability, detersive properties and mildness to human skin. Surprisingly, the resultant homogeneous liquid detergent exhibits the same or better foam performance, both as to initial foam volume and stability of foam in the presence of soils, and cleaning efficacy as an anionic based light duty liquid detergent (LDLD).

The essential ingredients discussed above are solubilized in an aqueous medium comprising water and solubilizing ingredients including the essential alkyl monoethanol amide and alkyl diethanol amide. As alkyl monoethanol amides, C₁₂-C₁₄ alkyl monoethanol amide (LMMEA), may be cited and as alkyl diethanol amides, coco diethanol amide (CDEA) or lauryl diethanol amide (LDEA), may be cited. Other solubilizing ingredients comprise C₂-C₃ mono and di-hydroxy alkanols, e.g., ethanol, isopropanol and propylene glycol. Suitable water soluble hydrotropic salts include sodium, potassium, ammonium and mono-, di- and triethanolammonium salts. While the aqueous medium is primarily water, preferably said solubilizing agents are included in order to control the viscosity of the liquid composition and to control low temperature cloud clear properties. Usually, it is desirable to maintain clarity to a temperature in the range of 5°C to 10°C. Therefore, the proportion of solubilizer generally will be from 1%-15%, preferably 2%-12%, most preferably 3%-8%, by weight of the detergent composition with the proportion of ethanol, when present, being 5% of weight or less in order to provide a composition having a flash point above about 46°C. Preferably the solubilizing ingredient will be a mixture of ethanol and either sodium xylene sulfonate or sodium cumene sulfonate or a mixture of said sulfonates or ethanol and urea. Inorganic salts such as sodium sulfate, magnesium sulfate, sodium chloride and sodium citrate can be added at concentrations of 0.5 to 4.0 wt.% to modify the cloud point of the nonionic surfactant and thereby control the haze of the resultant solution. Various other ingredients such as urea at a concentration of 0.5 to 4.0 wt.% or urea at the same concentration in combination with ethanol at a concentration of 0.5 to 4.0 wt.% can be used as solubilizing agents. Other ingredients which have been added to the compositions at concentrations of 0.1 to 4.0 wt. percent are perfumes, sodium bisulfite, ETDA, isoethanoeic and proteins such as lexine protein.

The foregoing solubilizing ingredients also facilitate the manufacture of the inventive compositions because they tend to inhibit gel formation.

In addition to the previously mentioned essential and optional constituents of the light duty liquid detergent, one may also employ normal and conventional adjuvants, provided they do not adversely affect the properties of the detergent. Thus, there may be used various coloring agents and perfumes; products of GAF Corporation; sequestering agents such as ethylene diamine tetraacetates; magnesium sulfate heptahydrate; pearlescing agents and opacifiers; pH modifiers; etc. The proportion of such adjuvant materials, in total will normally not exceed 15% of weight of the detergent composition, and the percentages of most of such individual components will be a maximum of 5% by weight and preferably less than about 2% by weight. Sodium bisulfite can be used as a color stabilizer at a concentration of 0.01 to 0.2 wt.%

The present nonionic based light duty liquid detergents such as dishwashing liquids are readily made by simple mixing methods from readily available components which, on storage, do not adversely affect the entire composition. However, it is preferred that the nonionic surfactant be mixed with the solubilizing ingredients, e.g., ethanol and, if present, prior to the addition of the water to prevent possible gelation. The nonionic based surfactant system is prepared by sequentially adding with agitation the anionic surfactant and the betaine to the non-ionic surfactant which has been previously mixed with a solubilizing agent such as LMMEA, LDEA and/or CDEA to assist in solubilizing said surfactants, and then adding with agitation the formula amount of water to form an aqueous solution of the nonionic based surfactant system. The use of mild heating (up to 100°C.) assists in the solubilization of the surfactants. The viscosities are adjustable by changing the total percentage of active ingredients. Usually, no thickening agent is added, but thickeners may be added if higher viscosity liquids are desired. In all such cases the product made will be pourable from a relatively narrow mouth bottle (1.5 cm. diameter) or opening, and the viscosity of the detergent formulation will not be so low as to be like water. The viscosity of the detergent desirably will be at least 100 centipoises (cps) at room temperature, but may be up to about 800 centipoises as measured with a Brookfield Viscometer using a number 2 spindle rotating at 30 rpms more preferably 200 to 600 cps and most preferably 250 to 450 cps. Its viscosity may approximate those of commercially acceptable detergents now on the market. The detergent viscosity and the detergent itself remain stable on storage for lengthy periods of time, without color changes or settling out of any insoluble materials. The pH of this formation is substantially neutral to skin, e.g., 4.5 to 8 and preferably about 5.5.

These products have unexpectedly desirably properties. For example, the foam quality and detersive property is equal to or better than standard light duty liquid detergents while using a nonionic surfactant as the primary surfactant and minimal amounts of anionic surfactant, thereby achieving a mild, non-irritating liquid detergent.

The following examples which are made by the previously described simple mixing, procedure are merely illustrative of the invention.

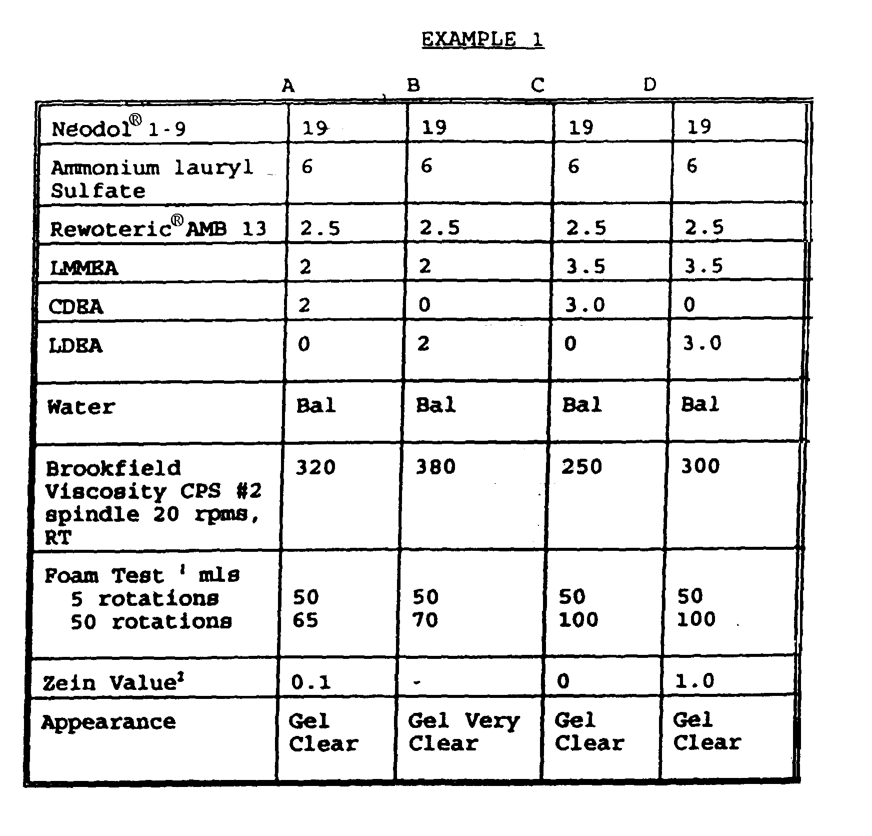

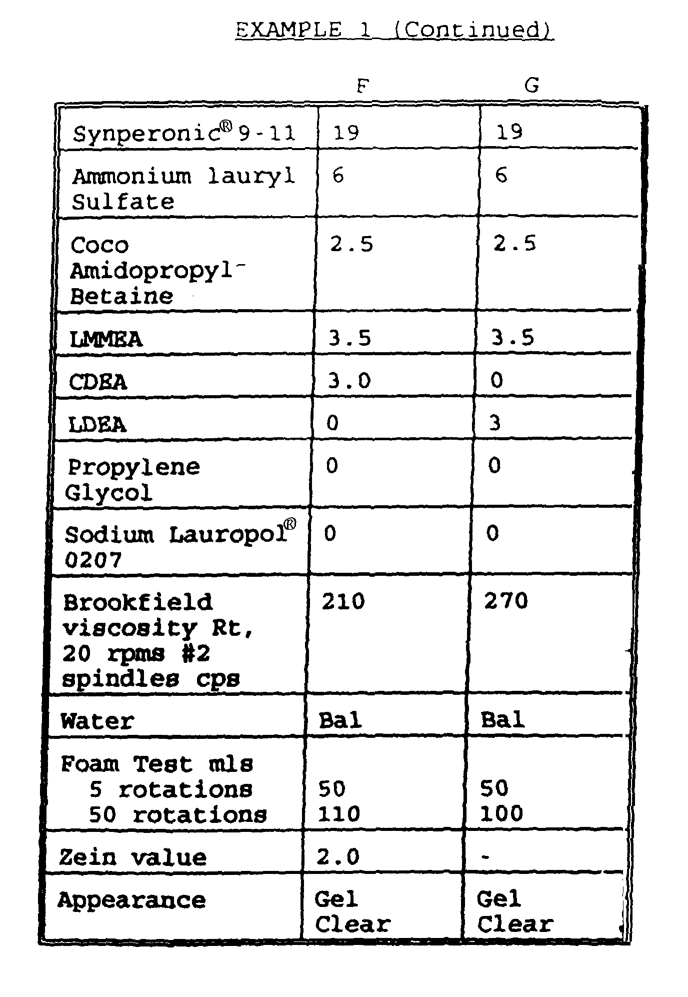

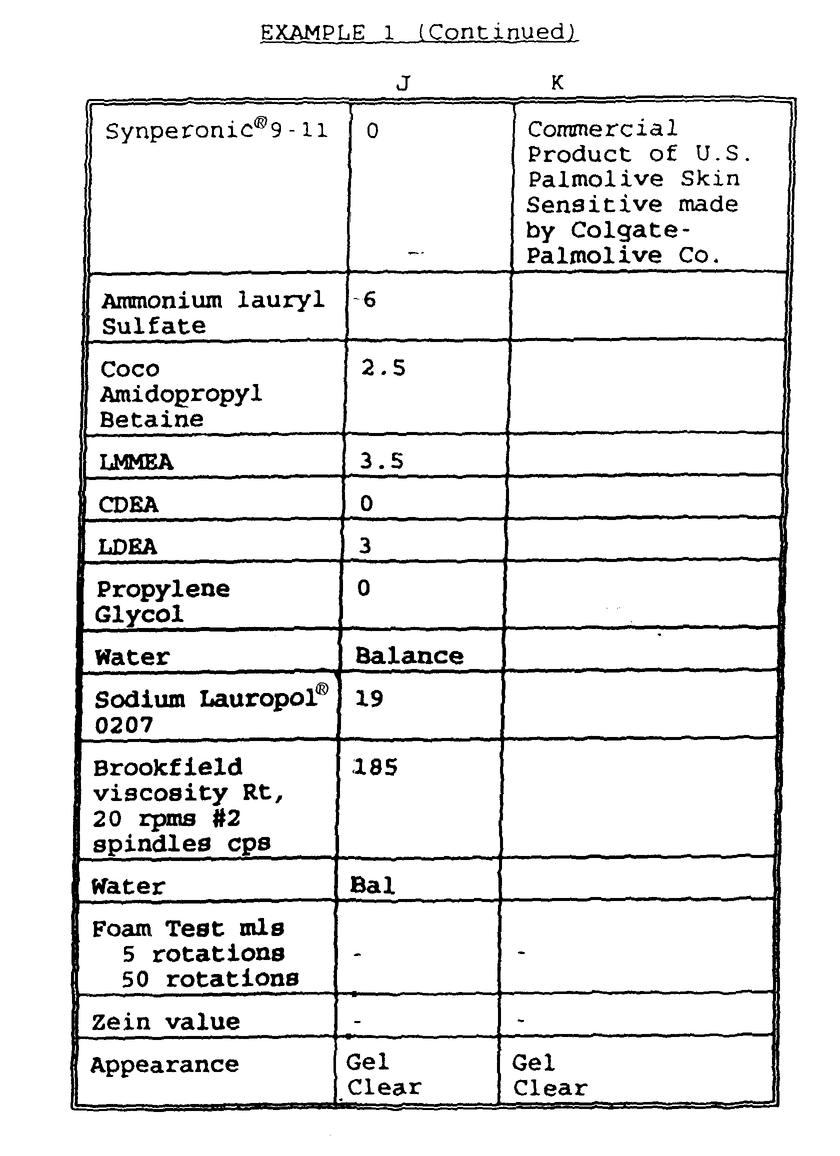

### FOAM VOLUME PROCEDURE

Using rotating cylinders we use 100 ml of an LDLD solution. Some corn oil is added to this solution. We start with the rotation of the cylinders. Every 5 rotations, the foam volume is recorded and finally after 50 rotation the foam volume is reported. The medium amount of foam after 5 rotations is about 25 mls, and more preferably about 40 mls an the minimum foam value after 50 rotations is about 40 mls and more preferably 50 mls.

### ZEIN TEST OR PROTEIN SOLUTIONIZATION TEST

A 1.0 percent LDLD solution is prepared, and Zein protein is added to this solution. After one hour the extra amount of protein is removed and the amount of protein dissolved it is evaluated. The higher the Zein figure is, the less mild the product is. The maximum zein value is about 10, more preferably 8 mg Zein/ml 1.0 percent solution.

### EXAMPLE 2

The following formulas were prepared to the previously devinded process.

| | A | B | C | D |
|---|---|---|---|---|
| Neodel® 1-9 | 19 | 19 | 19 | 19 |
| Ammonia Lauryl Sulfate | 6 | 6 | 6 | 6 |
| Rewoteric® AMB 13 | 2.5 | 2.5 | 2.5 | 2.5 |
| LMMEA | 3.5 | 3.5 | 3.5 | 3.5 |
| LDEA | 3.0 | 3.0 | 3.0 | 3.0 |
| NaC1 | 2.0 | 0 | 0 | 0 |
| MgS0₄ | 0 | 2.0 | 0 | 0 |
| NaCitrate | 0 | 0 | 2.0 | 0 |
| Urea | 0 | 0 | 2.0 | 0 |
| Na2So₄ | 0 | 0 | 0 | 2 |
| Na Bisulfate | 0.05 | 0.05 | 0.5 | 0.05 |
| ETDA | 0.045 | 0.045 | 0.045 | 0.045 |
| Perfume | 0.2 | 0.2 | 0.2 | 0.2 |
| Water | Balance | Balance | Balance | Balance |
| Brookfield viscosity cps RT, #2 spindle, 20 rpms | 350 | 370 | 330 | 200 |
| Foam Test mls | | | | |
| 5 rotations | 50 | - | - | - |
| 50 rotations | 100 | - | - | - |
| Zein value | 2.0 | - | - | - |
| Appearance | Gel/Clear | Gel/Clear | Gel/Clear | Gel/Clear |

## Claims

1. A high foaming, nonionic surfactant-based, light duty, liquid detergent comprising, by weight,
(a) 10% to 30% of a water soluble nonionic surfactant selected from the group consisting of primary and secondary C₈-C₁₈ alkanol condensates with 5 to 30 moles of ethylene oxide, condensates of C₈-C₁₈ alkylphenol with 5 to 30 moles of ethylene oxide, condensates of C₈-C₂₀ alkanol with a heteric mixture of ethylene oxide and propylene oxide having a weight ratio of ethylene oxide to propylene oxide from 2.5:1 to 4:1 and a total alkylene oxide content of 60% to 85% by weight and condensates of 2 to 30 moles of ethylene oxide with sorbitan mono and tri- C₁₀-C₂₀ alkanoic acid esters having an HLB of 8 to 15;
(b) 1% to 10% of a water-soluble anionic detergent selected from the group consisting of C₈-C₁₈ alkyl sulfates, C₈-C₁₆ alkylbenzene sulfonates, C₁₀-C₂₀ paraffin sulfonates, C₁₀-C₂₄ alpha olefin sulfonates and C₈-C₁₈ alkyl sulfosuccinate esters, C₈-C₁₈ acyl isethionates and C₈-C₁₈ acyl taurates; and
(c) 0.5 % to 10 % of a water-soluble betaine;
(d) 0.1 to 5 wt. % of an alkyl monoethanol amide;
(e) 0.1 to 5.0 wt. % of an alkyl diethanol amide; and;
(f) balance being water as an aqueous medium in which said nononic surfactant, said anionic detergent and said betaine are solubilized in said water, wherein the sum of C and B being from 15% to 48% by weight of the composition of the total surfactant content, and said betaine is less than or equal to 2.5 wt.%, said nonionic surfactant being in excess of 50% by weight of the total surfactant content and said composition being free of anionic alkyl ether polyethenoxy sulfate detergent, formates, amine oxides and HEDTA.

2. A liquid detergent composition according to claim 1 which includes, in addition, 1% to 15% by weight of at least one ingredient selected from the group consisting essentially of alkali metal salts, alkaline earth metal salts, urea, aliphatic alcohols, proteins, isoethanoic and propylene glycol, mono- and di-hydroxy alkanols, water soluble salts of C₁-C₃ substituted benzene sulfonate hydrotropes and mixtures thereof.

3. A liquid detergent composition according to claim 1 further including ethanol in the amount of 5% by weight or less.

4. A liquid detergent composition according to claim 1 wherein said nonionic surfactant is said condensate of a primary C₈-C₁₈ alkanol with 5-30 moles of ethylene oxide.

5. A liquid detergent composition according to claim 4 wherein said anionic detergent is selected from the group consisting of C₁₂-C₁₆ alkyl sulfates, C₁₀-C₁₅ alkylbenzene sulfonates, C₁₃-C₁₇ paraffin sulfonates and C₁₂-C₁₈ alpha olefin sulfonates.

6. A liquid detergent composition according to claim 1 wherein said nonionic surfactant is present in an amount of 13 % to 25 % by weight, said anionic detergent is present in an amount of 2% to 9% by weight and said betaine is present in an amount of 2 to 9 % by weight. of 2% to 9% by weight and said betaine is present in an amount of 0.5 to 2.5% by weight.

7. A liquid detergent composition according to claim 6 wherein said anionic detergent is a C₁₂-C₁₆ alkyl sulfate.

8. A liquid detergent composition according to Claim 1 further including a perservative.

9. A liquid detergent composition according to Claim 1 further including a color stabilizer.

10. A method of preparing the liquid detergent of claim 2 which comprises the steps of first mixing said nonionic surfactant with the solubilizing agent, sequentially adding with agitation said anionic surfactant and said betaine and lastly adding with agitation, the formula amount of water to form an aqueous solution of the nonionic based surfactant composition.

## Patentansprüche

1. Stark schäumendes, flüssiges Feinreinigungsmittel auf Basis von nichtionischem Tensid, welches, bezogen auf das Gewicht, ungefähr
(a) 10% bis 30% von in Wasser löslichem nichtionischem Tensid ausgewählt aus der Gruppe bestehend aus kondensaten von primärem und sekundärem C₈-C₁₈ Alkanol mit 5 bis 30 Molen Ethylonoxid, Kondonsaten von C₈-C₁₈ Alkylphenol mit 5 bis 30 Molen Ethylenoxid, Kondensaten von C₈-C₂₀ Alkanol mit einer heterischen Mischung von Ethylenoxid und Propylenoxid mit einem Gewichtsverhältnis von Ethylenoxid zu Propylenoxid von 2,5:1 bis 4:1 und einem Gesamtalkylenoxidgehalt von 60 Gew.% bis 85 Gew.% und Kondensaten von 2 bis 30 Molen Ethylenoxid mit Sorbitanmono- und tri-C₁₀-C₂₀-alkansäureestern mit einem HLB-Wert von 8 bis 15,
(b) 1% bis 10% von in Wasser löslichem anionischem Reinigungsmittel ausgewählt aus der Gruppe bestehend aus C₈-C₁₈ Alkylsulfaten, C₈-C₁₆ Alkylbenzolsulfonaten, C₁₀-C₂₀ Paraffinsulfonaten, C₁₀-C₂₄ α-Olefinsulfonaten und C₈-C₁₈ Alkylsulfosuccinatestern, C₈-C₁₈ Acylisethionaten und C₈-C₁₈ Acyltauraten, und
(c) 0,5% bis 10% von in Wasser löslichem Betain,
(d) 0,1 bis 5 Gew.% von Alkylmonoethanolamid,
(e) 0,1 bis 5,0 Gew.% von Alkyldiethanolamid, und
(f) als Rest Wasser als wäßriges Medium umfaßt, in dem das nichtionische Tensid, das anionische Reinigungsmittel und das Betain solubilisiert sind,
wobei die Summe von (c) und (b) 15 bis 48 Gew.% des gesamten Tensidgehalts ausmacht, und das Betain weniger als oder gleich 2,5 Gew.% und das nichtionische Tensid mehr als 50 Gew.% des gesamten Tensidgehalts ausmachen und die Zusammensetzung frei von anionischem Alkyletherpoly-ethenoxysulfatreinigungsmittel, Formiaten, Aminoxiden und HEDTA ist.

2. Flüssige Reinigungsmittelzusammensetzung nach Anspruch 1, die außerdem 1 bis 15 Gew.% von mindestens einem Bestandteil, ausgewählt aus der Gruppe bestehend im wesentlichen aus Alkalimetallsalzen, Erdalkalimetallsalzen, Harnstoff, aliphatischen Alkoholen, Proteinen, Isoethanoeic und Propylenglykol, Mono- und Dihydroxyalkanolen, in Wasser löslichen Salzen von C₁-C₃ substituierten Benzolsulfonat-Hydrotropen und Mischungen derselben.

3. Flüssige Reinigungsmittelzusammensetzung nach Anspruch 1, die ferner Ethanol in einer Menge von 5 Gew.% oder weniger umfaßt.

4. Flüssige Reinigungsmittelzusammensetzung nach Anspruch 1, bei der das nichtionische Tensid das Kondensat eines primären C₈-C₁₈ Alkanols mit 5 bis 30 Molen Ethylenoxid ist.

5. Flüssige Reinigungsmittelzusammensetzung nach Anspruch 4, bei der das anionische Reinigungsmittel ausgewählt ist aus der Gruppe bestehend aus C₁₂-C₁₆ Alkylsulfaten, C₁₀-C₁₅ Alkylbenzolsulfonaten, C₁₃-C₁₇ Paraffinsulfonaten und C₁₂-C₁₈ α-Olefinsulfonaten.

6. Flüssige Reinigungsmittelzusammensetzung nach Anspruch 1, bei der das nichtionische Tensid in einer Menge von 13 bis 25 Gew.% vorhanden ist, das anionische Reinigungsmittel in einer Menge von 2 bis 9 Gew.% vorhanden ist und das Betain in einer Menge von 2 bis 9 Gew.% vorhanden ist.

7. Flüssige Reinigungsmittelzusammensetzung nach Anspruch 6, bei der das anionische Reinigungsmittel ein C₁₂-C₁₆ Alkylsulfat ist.

8. Flüssige Reinigungsmittelzusammensetzung nach Anspruch 1, die ferner Konservierungsmittel umfaßt.

9. Flüssige Reinigungsmittelzusammensetzung nach Anspruch 1, die ferner Farbstabilisator umfaßt.

10. Verfahren zur Herstellung des flüssigen Reinigungsmittels gemäß Anspruch 2, bei dem zuerst das nichtionische Tensid mit dem Solubilisierungsmittel gemischt wird, nacheinander unter Bewegung das anionische Tensid und das Betain zugesetzt werden und schließlich unter Bewegung die Formelmenge Wasser zugesetzt wird, um eine wäßrige Lösung der auf nicht ionischem Tensid basierenden Tensidzusammensetzung zu bilden.

## Revendications

1. Un détergent liquide très moussant pour lavage délicat à base d'agent tensio-actif non ionique, comprenant approximativement, en poids,
(a) 10 % à 30 % d'un agent tensio-actif non ionique hydrosoluble choisi dans le groupe formé par les produits de condensation d'alcanol en C₈-C₁₈ primaire ou secondaire avec 5 à 30 moles d'oxyde d'éthylène, les produits de condensation de (alkyle en C₈-C₁₈)phénol avec 5 à 30 moles d'oxyde d'éthylène, les produits de condensation d'alcanol en C₈-C₂₀ avec un mélange hétérogène d'oxyde d'éthylène et d'oxyde de propylène ayant un rapport en poids de l'oxyde d'éthylène à l'oxyde de propylène de 2,5:1 à 4:1 et une teneur totale en oxydes d'alkylène de 60 % à 85 % en poids, et les produits de condensation de 2 à 30 moles d'oxyde d'éthylène avec des mono- et triesters d'acides alcanoïques en C₁₀-C₂₀ de sorbitanne ayant un RHL de 8 à 15 ;
(b) 1 % à 10 % d'un détergent anionique hydrosoluble choisi dans le groupe formé par les alkylsulfates en C₈-C₁₈, les (alkyle en C₈-C₁₆)benzène-sulfonates, les paraffine-sulfonates en C₁₀-C₂₀, les alpha-oléfine-sulfonates en C₁₀-C₂₄ et les esters d'alkyle en C₈-C₁₈ de sulfosuccinates, les (acyle en C₈-C₁₈)iséthionates et les (acyle en C₈-C₁₈)-taurinates ; et
(c) 0,5 % à 10 % d'une bétaïne hydrosoluble ;
(d) 0,1 à 5 % en poids d'un alkylmonoéthanolamide ;
(e) 0,1 à 5,0 % en poids d'un alkyldiéthanolamide ; et
(f) le reste d'eau sous forme d'un milieu aqueux dans lequel ledit agent tensio-actif non ionique, ledit détergent anionique et ladite bétaïne sont solubilisés dans ladite eau, la somme de C et B étant de 15 % à 48 % en poids de la teneur totale en agents tensio-actifs de la composition, et la proportion de ladite bétaïne étant inférieure ou égale à 2,5 % en poids, ledit agent tensio-actif non ionique étant présent en excès de 50 % en poids de la teneur totale en agents tensio-actifs, et ladite composition ne contenant pas de détergent anionique du type alkyl-polyoxyéthylène-sulfate, de formiates, d'oxydes d'amine et de HEDTA.

2. Une composition détergente liquide selon la revendication 1, qui contient, de plus, 1 % à 15 % en poids d'au moins un ingrédient choisi dans le groupe formé essentiellement par les sels de métaux alcalins, les sels de métaux alcalino-terreux, l'urée, les alcools aliphatiques, les protéines, isoéthanoïque et le propylène-glycol, les alcanols mono- et dihydroxylés, les sels hydrosolubles hydrotropes du type benzènesulfonate à substitution en C₁-C₃, et leurs mélanges.

3. Une composition détergente liquide selon la revendication 1, contenant, de plus, de l'éthanol en une proportion de 5 % en poids ou moins.

4. Une composition détergente liquide selon la revendication 1, dans laquelle ledit agent tensio-actif non ionique est ledit produit de condensation d'un alcanol primaire en C₈-C₁₈ avec 5 à 30 moles d'oxyde d'éthylène.

5. Une composition détergente liquide selon la revendication 4, dans laquelle ledit détergent anionique est choisi dans le groupe formé par les alkylsulfates en C₁₂-C₁₆, les (alkyle en C₁₀-C₁₅)benzène-sulfonates, les paraffine-sulfonates en C₁₃-C₁₇ et les alpha-oléfine-sulfonates en C₁₂-C₁₈.

6. Une composition détergente liquide selon la revendication 1, dans laquelle ledit agent tensio-actif non ionique est présent en une proportion de 13 % à 25 % en poids, ledit détergent anionique est présent en une proportion de 2 % à 9 % en poids et ladite bétaïne est présente en une proportion de 2 à 9 % en poids.

7. Une composition détergente liquide selon la revendication 6, dans laquelle ledit détergent anionique est un alkylsulfate en C₁₂-C₁₆.

8. Une composition détergente liquide selon la revendication 1, contenant de plus un conservateur.

9. Une composition détergente liquide selon la revendication 1, contenant de plus un stabilisant de couleur.

10. Un procédé de préparation du détergent liquide de la revendication 2, qui comprend les étapes consistant à mélanger d'abord ledit agent tensio-actif non ionique avec l'ajent solubilisateur, ajouter successivement, sous agitation, ledit agent tensio-actif anionique et ladite bétaïne, et enfin ajouter, sous agitation, la quantité d'eau de la formule pour former une solution aqueuse de la composition à base d'agent tensio-actif non ionique.
